# EUROPEAN PATENT SPECIFICATION

(11) **EP 3 986 109 B1**
(45) Date of publication and mention of the grant of the patent: **14.01.2026**
(21) Application number: 20745274.9
(22) Date of filing: 21.06.2020
(51) Int. Cl.: A01C 1/00, A61L 2/00, A61L 2/14, A01C 1/08

(54) **PLASMA TREATMENT**
PLASMABEHANDLUNG
TRAITEMENT AU PLASMA

(30) Priority: 21.06.2019 GB 201908902
(43) Date of publication of application: 27.04.2022
(73) Proprietor: Zayndu Ltd, Loughborough, England, LE11 3QF (GB)
(72) Inventor: IZA, Felipe, Loughborough, Leicestershire LE11 3TU (GB); WRIGHT, Alexander R P, Loughborough, Leicestershire LE11 3TU (GB); SHAW, Alexander H, Loughborough, Leicestershire LE11 3TU (GB); SHAMA, Gilbert, Loughborough, Leicestershire LE11 3TU (GB)
(74) Representative: Barker Brettell LLP
(86) International application number: PCT/IB2020/055842
(87) International publication number: WO 2020/255100

(56) References cited:
- WO-A1-00/78124
- WO-A1-2012/112042
- WO-A1-2016/096751
- KR-B1- 101 976 164
- US-A1- 2016 227 699

## Description

This invention relates to apparatus and method for plasma treatment, typically of materials and in one embodiment seeds.

It is known that various seeds can be subject to infections from viruses, bacteria and fungi. An example of this is *Pastinaca sativa* (parsnip) seeds, which can be infected by, amongst other fungi, *Itersonilia pastinaceae.* It is desirable to be able to treat these infections. Typically, at present, such seeds are immersed in hot water, which is cumbersome, often ineffective and requires drying of the seeds afterwards, or are treated with high temperatures (e.g. steam-based process) or with harsh chemical treatments, all of which are undesirable.

More generally, it is desirable to provide an apparatus by means of which various materials could be treated.

We are aware of:
- WO00/78124, which discloses living matter such as seeds being treated by exposing the living matter to a cold plasma to form a plasma reacted deposit on the surfaces of the living matter. The system operates at around 200 milliTorr (0.00026 atm).
- WO2012/112042, which discloses a device for disinfecting plant seeds comprising at least one accommodating space for plant seeds to be disinfected, at least one plasma generating unit for generating a plasma extending to said at least one accommodating space, and at least one gas flow generating element for generating a gas flow causing at least partial displacement of the plasma generated by the plasma generating unit to surround the plant seeds substantially entirely by plasma.
- US2016/227699, which discloses methods and apparatuses to activate, modify, and sanitize the surfaces of granular, powdered, or seed material placed in a continuous flow of a low-temperature, reduced-pressure gas plasma. The plasma is generated at pressures in the 0.01 to 10 Torr range (0.000013 to 0.013 atm).
- KR 101 976 164, which discloses a plasma seed treatment apparatus, which comprises: a magnetron antenna in which microwaves are generated; a plurality of vacuum chambers in which the microwaves propagate; a plasma induction antenna disposed at the centre of the vacuum chambers to generate plasma; and a stirring chamber positioned inside the vacuum chamber and rotated to stir seeds.
- WO2016/096751, which discloses a thermal and non-thermal plasma activated water reactor system that includes a reaction chamber, where the reaction chamber includes a gas inlet, a water inlet, a gas and water outlet, a ground electrode and reaction electrodes, where the water inlet and the water outlet are disposed to form a water vortex in the reaction chamber when water flows there through, where the reaction electrodes include a thermal plasma electrode and a non-thermal plasma electrode, and a plasma activated water reservoir that is disposed to receive the plasma activated water from the reaction chamber and disposed to return the plasma activated water to the reaction chamber.

The invention aims to improve and simplify the known apparatuses and methods by proposing a plasma treatment apparatus according to claim 1 and a method of treating material according to claim 9.

Thus, the agitation apparatus can allow for the contents of the void to be agitated in reactive species produced from the ionised gas plasma produced by the source in order to treat those contents. This is useful, in particular, for the treatment of materials comprising many small components, such as seeds, granular material, plastic beads and the like. The material could otherwise comprise biomass, waste water, milk (for sterilisation thereof), or water or other material contaminated by pollutants.

Reactive species produced from the ionised gas plasma can be used to treat those materials, with the reactive species produced from the ionised gas plasma reaching more of the material than if the material were not agitated, or if there were not a source in communication with the void.

In the example of seeds, we have appreciated that the apparatus can be used for the disinfection of seeds, and in particular to kill bacteria, fungus or fungal spores on seeds. It can also be used to promote seed germination.

The source may be within the void; alternatively, it may be in communication with the void through a conduit for the ionised gas plasma and/or for reactive species generated from the ionised gas plasma.

The agitation apparatus may comprise part of the housing comprising at least one rotating wall. Each rotating wall may be arranged for rotation around the source.

The apparatus may comprise drive means arranged to rotate each rotating wall around the source. Thus, each rotating wall can be driven for rotation about the source, so as to cause the agitation described above. Typically, the drive means will comprise a motor, such as an electric motor. The drive means may also comprise a transmission member, such as a drive belt or drive gear, arranged to transmit rotational movement of a rotor of the motor to each rotating wall.

Typically the walls will comprise at least one fixed wall which is fixed relative to the source. The at least one rotating wall may form a tube having two ends, and there may be a fixed wall at each end. Typically, the at least one rotating wall forms a cylinder and there is a fixed wall of circular shape at each end. The apparatus may comprise a bearing at each end through which each rotating wall is mounted on the fixed wall at that end.

The apparatus may be provided with a power conduit for transmitting electric power to the source from outside of the void. Typically, the conduit may comprise an electrical connection passing through a fixed wall, typically the fixed wall at an end. Thus, the conduit can be fixed in position (which is convenient when dealing with electrical connections) whilst the drum rotates.

The apparatus may comprise a power supply for the source, in electrical communication with the source, typically through the power conduit.

The agitation apparatus may comprise at least one baffle for agitating material within the drum. Typically, each baffle will form at least part of a helix about an axis about which each rotating wall rotates. Alternatively, each baffle may be straight, and there may be a plurality of baffles potentially parallel to one another.

The apparatus may be provided with a gas conduit through which a gas can be introduced into the void. The apparatus may also be provided with a gas exhaust conduit through which the gas can be exhausted from the void. As such, a gas, such as atmospheric air, dry air or an argon/oxygen mixture can be passed over the material in the void, which can add to the treatment of the material. The gas may comprise water vapour, which can increase the humidity of the air or gas within the void.

The apparatus may be provided with a source of visible or ultra-violet radiation, arranged so as to provide the visible or ultra-violet radiation within the void. Thus, light or ultra-violet radiation can be additionally used to treat the material in the void.

The source may be arranged to generate the ionised gas plasma from atmospheric air. This is plentiful, and is a useful source of both oxygen and nitrogen gas as precursors to form reactive species. Other oxygen and nitrogen containing gas mixtures such as helium/oxygen and argon/oxygen admixtures may also be used. **In** these cases, the apparatus may comprise delivery means for delivering the gas mixture to the ionised gas plasma source, typically from re-usable or single use gas canisters.

The apparatus may comprise a source of water in communication with the void. Typically, the source of water would be arranged to deliver water to the void. The source of water may comprise a valve by means of which the flow of water can be controlled.

The source of water may comprise a source of plasma activated water. Alternatively, the water introduced into the void may be converted to plasma activated water by the action of the reactive species generated from the ionised gas plasma.

The source may comprise at least two electrodes. Each electrode may be elongate, having a length. The electrodes may form pairs; with two electrodes there will be a single pair. Each pair of electrodes may be positioned so as to be skew, so that the lengths of the electrodes are non-parallel but the electrodes are spaced apart. Each pair of electrodes will define a crossing zone between and around the points along the length of each electrode where the other electrode of each pair comes closest. The source may further comprise a voltage source arranged to apply a voltage between the electrodes. When this voltage is applied, ionised gas plasma will be produced in the crossing zone. The minimum distance between the electrodes may be between 10 micrometres and 30 millimetres, typically between 0.5mm and 1.5mm.

Each electrode may comprise a metallic member surrounded by an encapsulating dielectric shield, such as a glass tube or ceramic coating. This limits the discharge current although the same could be achieved by quickly pulsing the applied voltage.

Typically, the voltage applied to generate the plasma from the power supply will be at least 0.1 or 0.5 kilovolts (kV), 1kV, 5kV, 10kV, 20kV or 40kV. The voltage may be an alternating current (AC) voltage, having a frequency. The frequency may be between 50Hz and 2.45GHz, and the voltage may be modulated so as to comprise alternating plasma-on periods where the voltage varies at the frequency and plasma-off periods where the voltage does not vary and will typically be zero. This may minimize heating of the material treated and reduce power consumption. Alternatively, the voltage can be applied as pulses with a pulse width duration between 0.1 nanosecond and 1000 milliseconds.

Typically, the power supply will be arranged to draw electrical power to generate the plasma from mains, a low-voltage DC supply (e.g. USB charger), generator, or batteries.

Typically, the apparatus will be operable to produce ionised gas plasma at room temperature and pressure; according to the invention the apparatus is operable with the temperature of the gas from which the ionised gas plasma is formed between 0 and 50 degrees Celsius, typically between 10 and 40 degrees Celsius, potentially between 15 and 30 degrees Celsius, and at 1 atmosphere ± 0.2 atm, 0.1 atm, 0.05atm or 0.01atm. The source may provide reactive species formed from ambient air surrounding the ionised gas plasma source, without first heating, cooling, pressuring and/or depressurising the ambient air.

The reactive species may comprise ozone. The reactive species may additionally or alternatively comprise at least one of Nitric Oxide (NO), Hydrogen Peroxide (H₂O₂), Hydroxyl radicals (OH), Hydron ions (H⁺), Nitrate ions (NO₃⁻), Nitrite ions (NO₂⁻) and Oxygen atoms (O).

According to a second aspect of the invention, there is provided a method of treating a material, comprising placing the material within the void of an apparatus in accordance with the first aspect of the invention, and then causing the source of ionised gas plasma to produce ionised gas plasma in the void whilst also causing each rotating wall to rotate about the source.

Thus, this aspect describes how the apparatus of the first aspect can be used to treat a material. Typically, the ionised gas plasma will generate reactive species which will accumulate in the void, through which the material will be tumbled by the action of the rotating walls.

Reactive species produced from the ionised gas plasma can be used to treat the material, with the reactive species produced from the ionised gas plasma reaching more of the material than if the walls did not rotate to tumble the material, or if the source was not within the void.

This is useful, in particular, where the material comprises many small components; as such the material may comprise seeds, granular material, plastic beads or the like. The material could otherwise comprise biomass, waste water, milk (for sterilisation thereof), or water or other material contaminated by pollutants.

In the example of seeds, we have appreciated that the method can be used for the disinfection of seeds, and in particular to kill bacteria, fungus or fungal spores on seeds. It can also be used to promote seed germination.

The method may comprise driving the rotating walls around the source using the drive means.

The method may comprise introducing a gas into the void through the gas conduit, and optionally exhausting the gas through the gas exhaust conduit. The gas may comprise atmospheric air, dry air or an argon/oxygen mixture. As such, a gas can be passed over the material in the void, which can add to the treatment of the material. The gas may comprise water vapour, which can increase the humidity of the air or gas within the void.

The method may comprise irradiating the material with visible or ultra-violet radiation.

Typically, the ionised gas plasma will be produced from ambient air at room temperature and pressure; that is with the temperature of the air from which the ionised gas plasma is formed between 0 and 50 degrees Celsius, and at 1 atmosphere ± 0.2 atm, 0.1 atm, 0.05atm or 0.01atm. The ionised gas plasma may be formed in ambient air surrounding the ionised gas plasma source, without first heating, cooling, pressuring and/or depressurising the ambient air.

The reactive species may comprise ozone. The reactive species may additionally or alternatively comprise at least one of Nitric Oxide (NO), Hydrogen Peroxide (H₂O₂), Hydroxyl radicals (OH), Hydron ions (H⁺), Nitrate ions (NO₃⁻), Nitrite ions (NO₂⁻) and Oxygen atoms (O).

There now follows, by way of example only, description of embodiments of the invention described with reference to the accompanying drawings, in which:
**Figure 1** shows a perspective view of an apparatus in accordance with a first embodiment of the invention;
**Figure 2** shows a part-sectional perspective view of the apparatus of Figure 1;
**Figure 3** shows an elevation of the apparatus of Figure 1;
**Figure 4** shows a plan view of the electrodes of the apparatus of Figure 1;
**Figure 5** shows a side-view cross-section of the electrodes of Figure 4;
**Figure 6** shows a graph demonstrating the fungus-killing properties of the apparatus of Figure 1;
**Figure 7** shows a graph demonstrating the effect that the apparatus of Figure 1 has on the germination of seeds;
**Figures** 8 **to 12** show alternative embodiments of the electrodes of the apparatus of Figure 1; and
**Figure 13** shows schematically an apparatus in accordance with a seventh aspect of the invention.

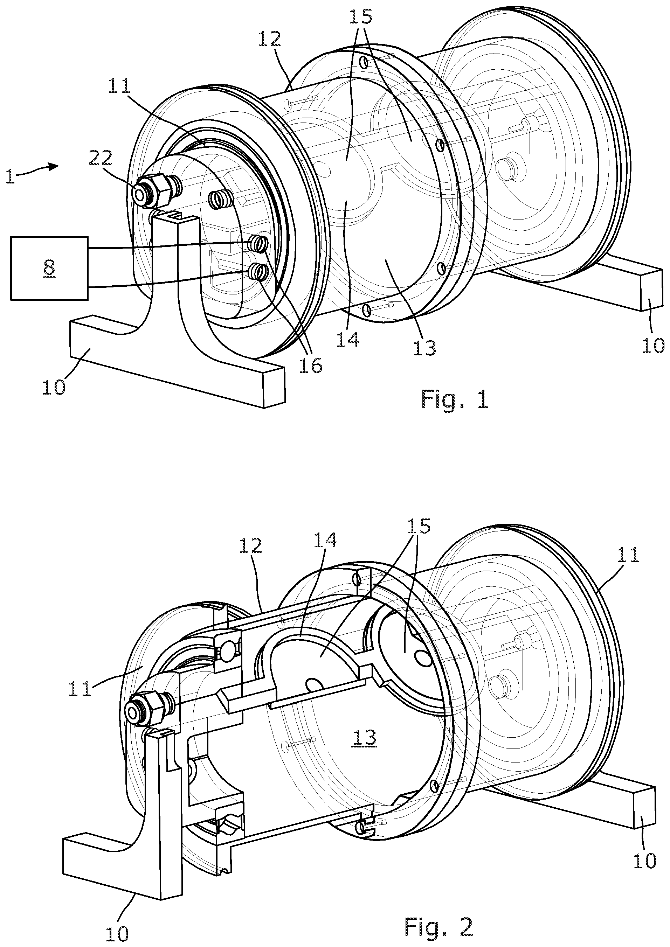

Figures 1 to 5 of the accompanying drawings show an apparatus 1 of the form of a reactor in accordance with a first embodiment of the present invention. The apparatus 1 comprises a base 10 on which are mounted two fixed disc-shaped end walls 11 at opposing ends of the apparatus.

Separating the end walls 11 there is a rotatable cylindrical wall 12. This can rotate about the axis of the cylinder it forms relative to the fixed end walls 11. Whilst in this embodiment a single cylindrical rotating wall 12 is provided, there need not be a single rotating wall, nor need it be cylindrical - for example, any prismoidal shape could be used, such as a hexagon made up of six walls, one for each face.

The fixed end walls 11 and the rotatable cylindrical wall 12 define a void 13. Within this void is provided a source of ionised gas plasma 14. The source 14 is mounted on the fixed end walls 11 so that the rotatable wall 12 is free to rotate around it.

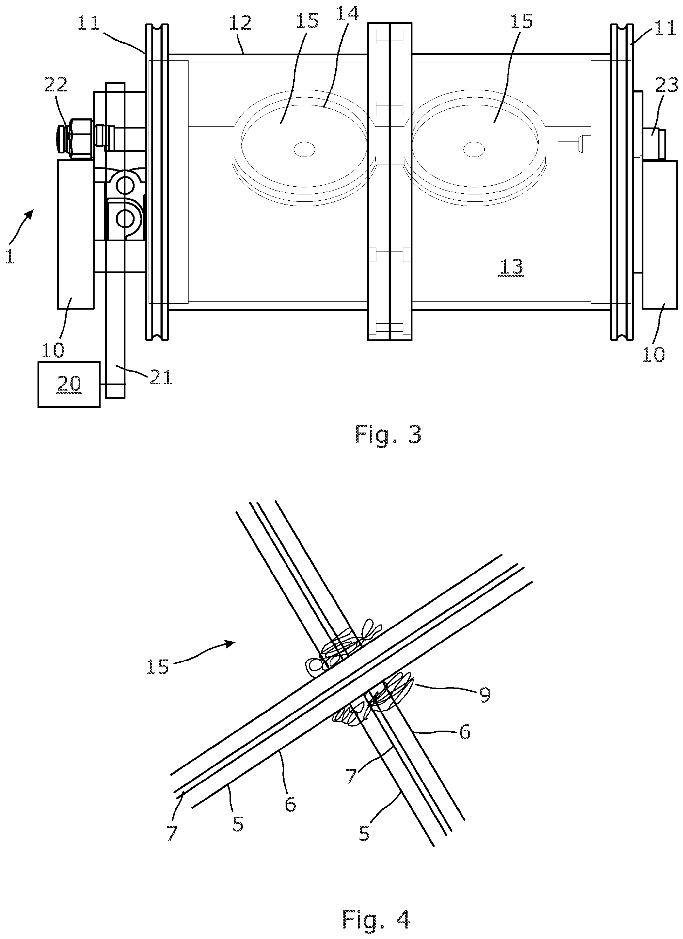

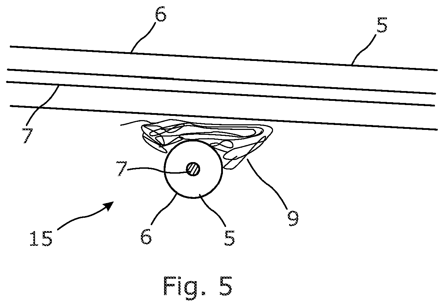

The source 14 comprises two sets of electrodes 15. One set of electrodes 15 is shown in Figures 4 and 5 of the accompanying drawings. Each set of electrodes 15 comprises a pair of electrodes 5 being connected to an output stage of a power supply 8 through electrical connections 16 through one fixed wall 11. Each electrode 5 comprises a central conducting wire 7, surrounded by a dielectric tube 6. The wire 7 is typically metallic, but could comprise graphite, stainless steel, copper, a conductive liquid or water, or any other suitable conductor; the dielectric is typically borosilicate glass, but could comprise polytetrafluoroethylene (PTFE) or other plastics, ceramics, alumina, Macor (RTM) or other machineable glass-ceramic mixtures, reinforced glass fibre or quartz, or any other suitable dielectric.

The two electrodes 5 are provided so their lengths are parallel to a common plane. However, the electrodes 5 are skew to each other, in that they are non-parallel but do not intersect, being spaced apart from each other vertically; in plan view (as shown in Figure 4 of the accompanying drawings) they form an X-shape, with only a small gap (1 mm) where they cross.

The power supply 8 is arranged to provide a voltage between the two electrodes 5. Typically, the voltage will be a sinusoidal excitation wave of around 20 kilovolts (kV) peak to peak at a frequency of around 30kHz. The signal can be modulated to comprise pulses having a 5% duty cycle (so each cycle would comprise 5% of the cycle with the voltage comprising a 20kV sinusoidal signal and the other 95% essentially at zero voltage).

When this voltage is applied between the electrodes, an ionised gas plasma 9 is formed in the region where the electrodes 5 cross, from the ambient air at the local temperature and pressure. This ionised gas plasma 9 then generates reactive species from air such as Nitric Oxide (NO), Ozone (O₃), Hydrogen Peroxide (H₂O₂), Hydroxyl radicals (OH), Hydron ions (H⁺), Nitrate ions (NO₃⁻), Nitrite ions (NO₂⁻) and Oxygen atoms (O). The ionised gas plasma can also generate ultraviolet (UV) radiation. These reactive species 9 diffuse throughout the void 13.

An electric motor 20 is provided which is arranged to rotate the rotatable wall 12 about its axis by means of drive belt 21. As such, the rotating walls provide an agitation apparatus for the contents of the void 13.

A gas inlet 22 is provided in one fixed wall 11 and a gas outlet 23 is provided in the other fixed wall 11. As such, gas, such as humidified air, can be introduced at one end of the void 13, passed over material in the void, and exhausted at the other end.

As such, in use, the reactive species are generated by the plasma generated by the source 14. These diffuse throughout the void. Material - particular material made up of many small parts, or liquid - will be tumbled through the reactive species in order to react with those reactive species. This tumbling effect can be increased by the use of linear or helical baffles on the inner wall of the rotatable wall 12.

This is useful, in particular, for the treatment of materials comprising many small components, such as seeds, granular material, plastic beads and the like. The material could otherwise comprise biomass, waste water, milk (for sterilisation thereof), or water or other material contaminated by pollutants.

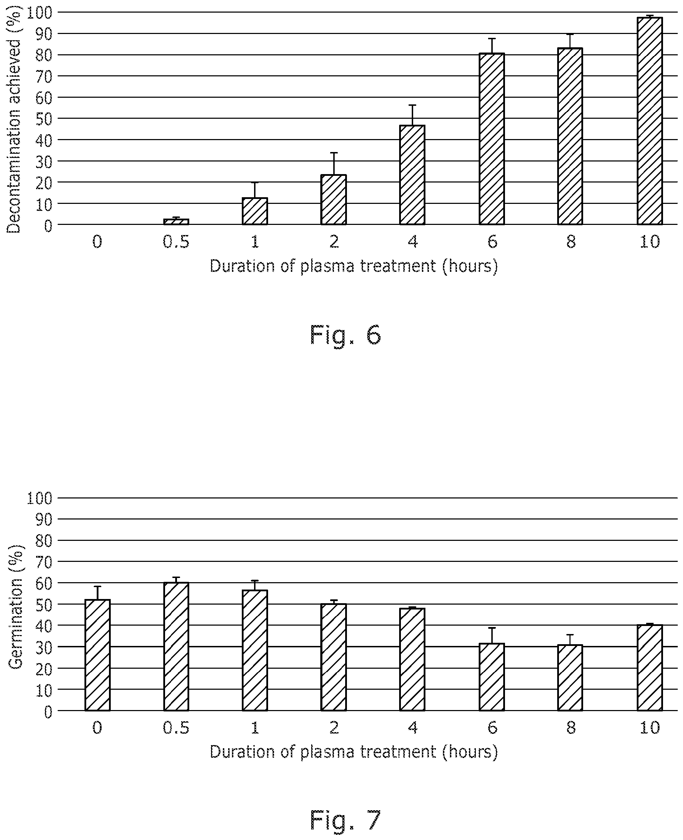

In the particular example of seeds, we have found that this can have an effect of killing fungal infections of seeds. Figure 6 of the accompanying drawings show the effect of the use of the apparatus 1 on parsnip seeds (*Pastinaca sativa).* The seeds had been contaminated with *Itersonilia.* The seeds were placed in the reactor, and the apparatus activated for between zero and ten hours as shown in the graph in Figure 6. One hundred of the seeds from each treatment time were then placed on agar with chloramphenicol and monitored for fungal growth after three days.

Figure 6 shows the percentage of seeds that were growth free after that time. It can be seen that increasing use of the apparatus increases the percentage of seeds that were free of fungal infection, and so indicates that the apparatus can be useful for killing this fungus on seeds. The error bars show the variability between the three times the experiment was run.

Figure 7 of the accompanying drawings shows the effect that the reactor has on germination rates. The same experiment as for Figure 6 was carried out and one hundred seeds per treatment placed on damp filter paper in petri dishes. The dishes were wrapped in parafilm and incubated at constant 24°C incubator with constant light. Figure 7 shows the percentage of seeds that had germinated (rupture of the testa and emergence of radicle) after 21 days.

As can be seen in Figure 7, there was no substantial decrease in germination rate, and indeed an increase in germination rate for exposure times of over six hours.

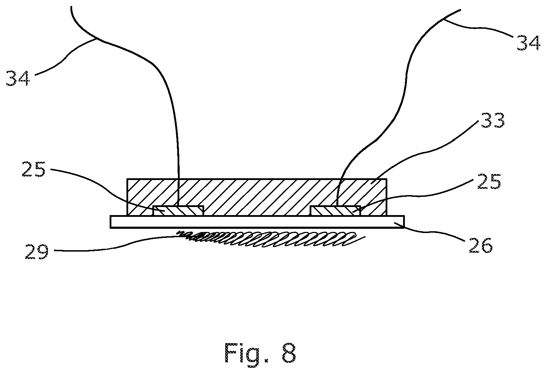

Alternative embodiments of the electrodes are shown in Figures 8 to 15 of the accompanying drawings. These could generally be used in place of the electrodes 15 shown above. The dielectric and electrode material can be as suggested above with respect to the first embodiment.

In the second embodiment of the invention shown in Figure 8, a dielectric plate 26 is used mounted within the void 13. On the upper surface of the dielectric plate are mounted two encapsulated parallel electrodes 25; wires 34 lead to a power supply (not shown) for the application of the voltage. The electrodes 25 are backed by resin insulation 33.

In this embodiment, application of the voltage will cause ionised gas plasma to be formed on the bottom surface of the dielectric plate 26, above the well 23. Reactive species generated in the plasma will then diffuse outwards into the void 13.

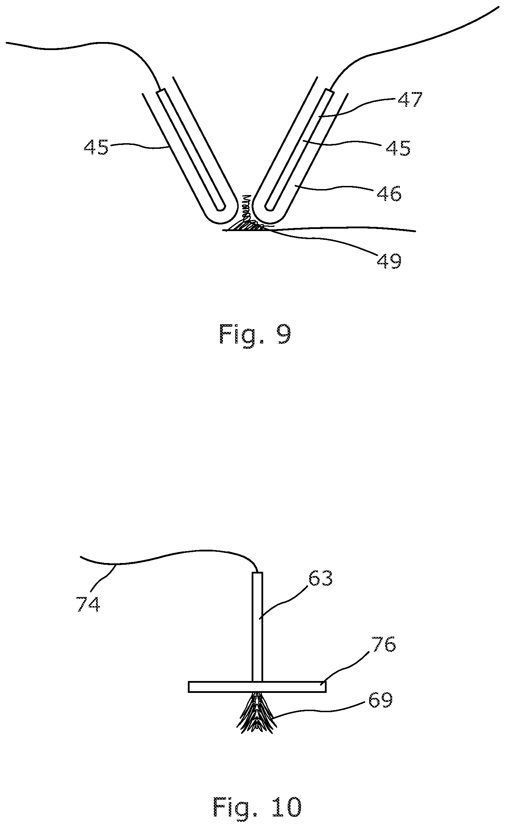

In the third embodiment of the invention shown in Figure 9 of the accompanying drawings, the reference numerals have been raised by 20 with respect to the second embodiment. In this embodiment, there are two electrodes 45 each comprising a central metallic rod 47 (with wires 54 to connect to the unshown power supply) surrounded by dielectric 46. The ends of the electrodes 45 are adjacent; on application of a voltage between the electrodes 45, plasma 49 will form between the ends of the electrodes 45 and reactive species generated in the plasma diffuse outwards into the void 13.

In the fourth embodiment of the invention shown in Figure 10 of the accompanying drawings, the reference numerals have been raised by 40 with respect to the second embodiment. In this embodiment, there is a single electrode 63 (with wire 74 to connect to the unshown power supply) terminated by a dielectric plate 26. A further electrode is provided elsewhere in the void 13. On application of a voltage between the electrodes, plasma 69 will form underneath the dielectric plate 26 beneath the electrode 63 and reactive species generated in the plasma diffuse outwards into the void 13.

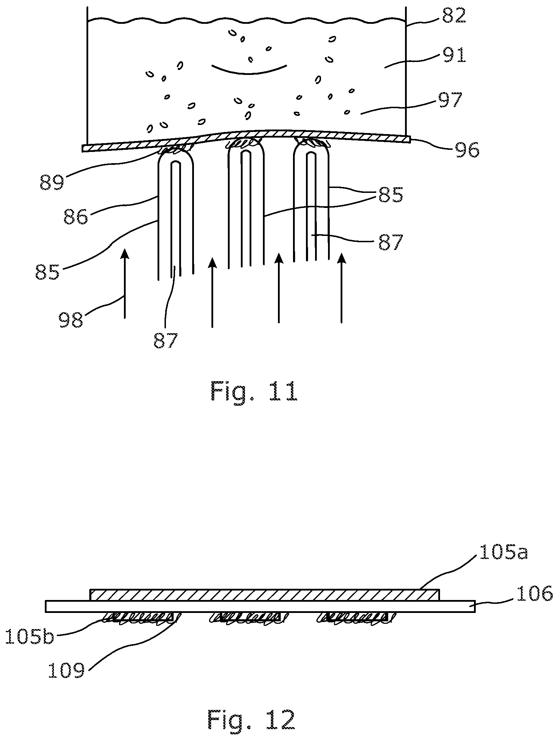

In the fifth embodiment of the invention shown in Figure 11 of the accompanying drawings, the reference numerals have been raised by 60 with respect to the second embodiment. In this embodiment, a well 82 contains an aqueous solvent 91 and as its bottom face has a porous membrane 96. Electrodes 85 are placed underneath, with their ends uppermost. Each electrode 85 comprises a central metallic (etc) rod 87 surrounded by a dielectric crucible. Air is passed upwards around the electrodes through the porous membrane to form bubbles 97 in the aqueous solvent.

**In** order to form a plasma 89 adjacent to the ends of the electrodes 85 and the porous membrane 96, the aqueous solvent can be used as another electrode as in the fifth embodiment (so that a wire would be placed in the conductive aqueous solvent 91) or the porous membrane could be conductive and used an electrode. Applying a voltage between either the membrane 96 or the aqueous solvent 91 on the one hand and the electrodes 85 on the other will cause a plasma to be formed adjacent to the underside of the porous membrane 96 and swept up with airflow 98 to form the bubbles 97. The content of the bubbles 97, including reactive species generated in the ionised gas plasma, will then dissolve in the aqueous solvent 91 to form plasma activated water, and/or will evolve out of the water into the void 13.

In the sixth embodiment of the invention shown in Figure 12 of the accompanying drawings, the reference numerals have been raised by 80 with respect to the second embodiment. In this embodiment, a dielectric sheet 106 separates two sets of electrodes 105a, 105b; a single planar electrode 105a on the top side of the dielectric sheet and a plurality of patch or elongate electrodes 105b on the bottom side. On application of a voltage between the electrodes 105a, 105b, plasma 109 will form around the electrodes 105b on the bottom side of the dielectric plate 106, and reactive species generated in the plasma diffuse outwards into the void.

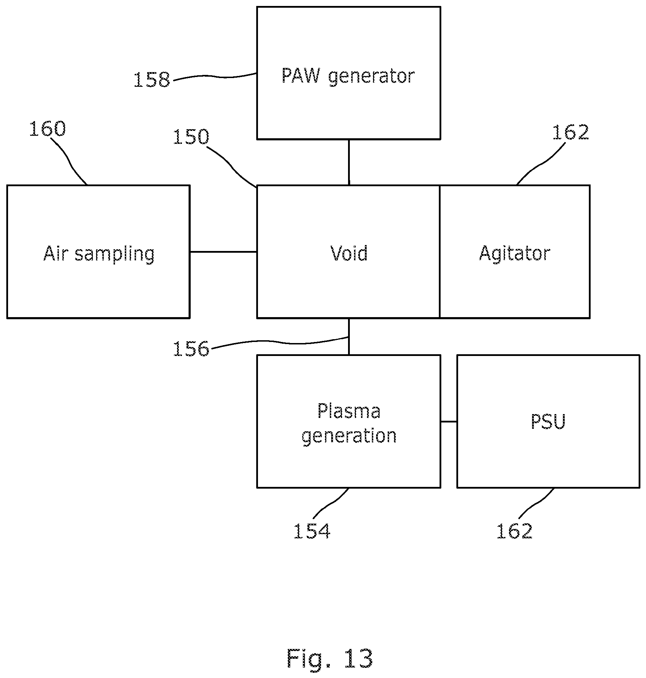

**In** the seventh apparatus of the invention shown in Figure 13 of the accompanying drawings, a void 150 is depicted which has an agitator 152 such as a vibrator, shaker or the like associated with it. The void can contain a material such as seeds or other particulate material to be treated.

A plasma generator 154 is connected to the void 150 through a conduit 156 (such as a pipe). As such, the plasma generator 154 generates ionised gas plasma from the surrounding air and passes that plasma, or more likely reactive species formed from the action of the ionised gas plasma in the air, through conduit 156 into the void in order to treat the material.

The apparatus further comprises a source 158 of plasma activated water (PAW). This can either be simple water, which is then passed into the void to be acted upon by reactive species there, or it can contain water that has already had such reactive species dissolved therein (for example, by the use of another source of ionised gas plasma).

An air sampling unit 160 is provided to determine the concentration of at least one reactive species within the void 150; the sampled air can be returned to the void 150 or vented elsewhere. The sampling unit 160 can then control a power supply unit (PSU) 112 for the plasma source 154 to control how much plasma and so home much of the reactive species is produced. This can form a system of closed-loop control.

## Claims

1. A plasma treatment apparatus, comprising:
• a housing (11, 12) defining a void (13);
• a source (14) of ionised gas plasma (9) in communication with the void (13); and
• agitation apparatus (12) arranged to agitate contents of the void (13)
**characterized in that** the apparatus is operable to produce ionised gas plasma (9) with the pressure of a gas from which the ionised gas plasma (9) is formed being 1 atmosphere ± 0.2 atm and **in that** a temperature of the gas from which the ionised gas is formed is between 0 and 50 degrees Celsius.

2. The apparatus of claim 1, in which the agitation apparatus (12) comprises part of the housing (11, 12) comprising walls comprising at least one rotating wall (12).

3. The apparatus of claim 1 or claim 2, in which the source (14) of ionised gas plasma (9) is within the void (13).

4. The apparatus of claim 3 as it depends from claim 2, in which each rotating wall is arranged for rotation around the source (14), the apparatus optionally comprising drive means (20, 21) arranged to rotate each rotating wall around the source (14).

5. The apparatus of claim 4, in which the walls comprise at least one fixed wall (11) which is fixed relative to the source (14) and the apparatus is optionally provided with a power conduit for transmitting electric power to the source (14) from outside of the void (13), comprising an electrical connection passing through a fixed wall.

6. The apparatus of any preceding claim, in which the agitation apparatus comprises at least one baffle arranged for movement, typically rotational, within the void (13).

7. The apparatus of any preceding claim, provided with a gas conduit (156) through which a gas can be introduced into the void (13) and optionally a gas exhaust conduit through which the gas can be exhausted from the void (13) and optionally a source of visible or ultra-violet radiation, arranged so as to provide the visible or ultra-violet radiation within the void (13).

8. The apparatus of any of claim 1 or claim 2, in which the source (14) is outside the void (13) and there is provided a conduit for the ionised gas plasma (9) or for reactive species generated by the ionised gas plasma (9) from the source (14) to the void (13), the apparatus optionally comprising a source of water in communication with the void (13), such as a source of plasma activated water.

9. A method of treating a material, comprising placing the material within the void (13) of an apparatus in accordance with any preceding claim, and then causing the source (14) of ionised gas plasma (9) to produce ionised gas plasma (9) whilst also causing the agitation apparatus to agitate the material.

10. The method of claim 9, in which the ionised gas plasma (9) generates reactive species which accumulate in the void (13), in which the reactive species optionally comprise at least one of ozone, Nitric Oxide (NO), Hydrogen Peroxide (H₂O₂), Hydroxyl radicals (OH), Hydron ions (H⁺), Nitrate ions (NO₃⁻), Nitrite ions (NO₂⁻) and Oxygen atoms (O).

11. The method of claim 9 or claim 10, in which the material comprises seeds.

12. The method of claim 9 or 10, in which the material comprises granular material, plastic beads, biomass, waste water, milk, or water or other material contaminated by pollutants.

13. The method of claim 11, in which the method results in the disinfection and/or cleaning of the seeds and/or the promotion of germination of the seeds, typically in which both the disinfection and/or cleaning of the seeds and the promotion of germination of the seeds occurs over the same period within which the seeds are not removed from the void (13).

14. The method of claim 11 or 13, in which the method results in the killing of viruses, bacteria, fungus or fungal spores on the seeds.

15. The method of any of claims 9 to 14, comprising introducing a gas into the void (13) through a gas conduit, and optionally exhausting the gas through the gas exhaust conduit and/or, where the apparatus comprises a source of water in communication with the void (13), adding water to the void (13) so as to generate plasma activated water within the void (13).

## Patentansprüche

1. Plasmabehandlungsvorrichtung, Folgendes umfassend:
• ein Gehäuse (11, 12), das einen Hohlraum (13) definiert;
• eine Quelle (14) von ionisiertem Gasplasma (9) in Kommunikation mit dem Hohlraum (13); und
• eine Rührvorrichtung (12), die angeordnet ist, um den Inhalt des Hohlraums (13) zu rühren,
**dadurch gekennzeichnet, dass** die Vorrichtung betreibbar ist, um ionisiertes Gasplasma (9) zu produzieren, wobei der Druck eines Gases, aus dem das ionisierte Gasplasma (9) gebildet wird, 1 Atmosphäre ± 0,2 atm beträgt, und dass die Temperatur des Gases, aus dem das ionisierte Gas gebildet wird, zwischen 0 und 50 Grad Celsius liegt.

2. Vorrichtung nach Anspruch 1, wobei die Rührvorrichtung (12) einen Teil des Gehäuses (11, 12) umfasst, das Wände umfasst, die mindestens eine rotierende Wand (12) umfassen.

3. Vorrichtung nach Anspruch 1 oder Anspruch 2, wobei sich die Quelle (14) von ionisiertem Gasplasma (9) innerhalb des Hohlraums (13) befindet.

4. Vorrichtung nach Anspruch 3 in Abhängigkeit von Anspruch 2, wobei jede rotierende Wand zum Rotieren um die Quelle (14) angeordnet ist, wobei die Vorrichtung optional ein Antriebsmittel (20, 21) umfasst, das angeordnet ist, um jede rotierende Wand um die Quelle (14) rotieren zu lassen.

5. Vorrichtung nach Anspruch 4, wobei die Wände mindestens eine fixierte Wand (11) umfassen, die relativ zu der Quelle (14) fixiert ist, und die Vorrichtung optional mit einer Stromleitung zum Übertragen von elektrischer Energie an die Quelle (14) von außerhalb des Hohlraums (13) bereitgestellt ist, umfassend eine elektrische Verbindung, die durch eine fixierte Wand verläuft.

6. Vorrichtung nach einem der vorhergehenden Ansprüche, wobei die Rührvorrichtung mindestens ein Leitblech umfasst, das innerhalb des Hohlraums (13) beweglich, typischerweise rotierend, angeordnet ist.

7. Vorrichtung nach einem der vorhergehenden Ansprüche, bereitgestellt mit einer Gasleitung (156), durch die ein Gas in den Hohlraum (13) eingeführt werden kann, und optional einer Gasablassleitung, durch die das Gas aus dem Hohlraum (13) abgelassen werden kann, und optional einer Quelle von sichtbarer oder ultravioletter Strahlung, die so angeordnet ist, dass sie die sichtbare oder ultraviolette Strahlung innerhalb des Hohlraums (13) bereitstellt.

8. Vorrichtung nach einem der Ansprüche 1 oder 2, wobei sich die Quelle (14) außerhalb des Hohlraums (13) befindet und eine Leitung für das ionisierte Gasplasma (9) oder für durch das ionisierte Gasplasma (9) erzeugte reaktive Spezies von der Quelle (14) zu dem Hohlraum (13) bereitgestellt ist, wobei die Vorrichtung optional eine Wasserquelle umfasst, die mit dem Hohlraum (13) in Kommunikation steht, wie etwa eine Quelle von plasmaaktiviertem Wasser.

9. Verfahren zum Behandeln eines Materials, umfassend das Platzieren des Materials in dem Hohlraum (13) einer Vorrichtung nach einem der vorhergehenden Ansprüche und das anschließende Veranlassen der Quelle (14) von ionisiertem Gasplasma (9), ionisiertes Gasplasma (9) zu produzieren, während des gleichzeitigen Veranlassens der Rührvorrichtung, das Material zu rühren.

10. Verfahren nach Anspruch 9, wobei das ionisierte Gasplasma (9) reaktive Spezies erzeugt, die sich in dem Hohlraum (13) ansammeln, wobei die reaktiven Spezies optional mindestens eines von Ozon, Stickoxid (NO), Wasserstoffperoxid (H₂O₂), Hydroxylradikalen (OH), Hydronionen (H⁺), Nitrationen (NO₃⁻), Nitritionen (NO₂⁻) und Sauerstoffatomen (O) umfassen.

11. Verfahren nach Anspruch 9 oder Anspruch 10, wobei das Material Samen umfasst.

12. Verfahren nach Anspruch 9 oder 10, wobei das Material Granulatmaterial, Kunststoffkügelchen, Biomasse, Abwasser, Milch oder Wasser oder anderes mit Schadstoffen kontaminiertes Material umfasst.

13. Verfahren nach Anspruch 11, wobei das Verfahren zur Desinfektion und/oder Reinigung der Samen und/oder zur Förderung der Keimung der Samen führt, typischerweise wobei sowohl die Desinfektion und/oder Reinigung der Samen als auch die Förderung der Keimung der Samen über den gleichen Zeitraum erfolgt, innerhalb dessen die Samen nicht aus dem Hohlraum (13) entfernt werden.

14. Verfahren nach Anspruch 11 oder 13, wobei das Verfahren zur Abtötung von Viren, Bakterien, Pilzen oder Pilzsporen auf den Samen führt.

15. Verfahren nach einem der Ansprüche 9 bis 14, umfassend das Einführen eines Gases in den Hohlraum (13) durch eine Gasleitung und optional das Ablassen des Gases durch die Gasablassleitung und/oder, wenn die Vorrichtung eine Wasserquelle in Kommunikation mit dem Hohlraum (13) umfasst, Zugeben von Wasser in den Hohlraum (13), um plasmaaktiviertes Wasser innerhalb des Hohlraums (13) zu erzeugen.

## Revendications

1. Appareil de traitement au plasma, comprenant :
• un boîtier (11, 12) définissant une cavité (13);
• une source (14) de plasma gazeux ionisé (9) en communication avec la cavité (13) ; et
• un appareil d'agitation (12) agencé pour agiter le contenu de la cavité (13),
**caractérisé en ce que** l'appareil est utilisable pour produire le plasma gazeux ionisé (9) avec une pression d'un gaz à partir duquel le plasma gazeux ionisé (9) est formé qui est de 1 atmosphère ± 0,2 atm, et **en ce que** la température du gaz à partir duquel le gaz ionisé est formé est comprise entre 0 et 50 degrés Celsius.

2. Appareil de la revendication 1, dans lequel l'appareil d'agitation (12) comprend une partie du boîtier (11, 12) comprenant des parois comprenant au moins une paroi rotative (12).

3. Appareil de la revendication 1 ou 2, dans lequel la source (14) de plasma gazeux ionisé (9) se situe à l'intérieur de la cavité (13).

4. Appareil de la revendication 3, telle qu'elle dépend de la revendication 2, dans lequel chaque paroi rotative est agencée pour tourner autour de la source (14), l'appareil comprenant éventuellement des moyens d'entraînement (20, 21) agencés pour faire tourner chaque paroi rotative autour de la source (14).

5. Appareil de la revendication 4, dans lequel les parois comprennent au moins une paroi fixe (11) qui est fixe par rapport à la source (14), et l'appareil est éventuellement muni d'un conduit d'alimentation destiné à transmettre de l'énergie électrique à la source (14) depuis l'extérieur de la cavité (13), comprenant une connexion électrique traversant une paroi fixe.

6. Appareil de l'une quelconque des revendications précédentes, dans lequel l'appareil d'agitation comprend au moins un déflecteur agencé pour effectuer un mouvement, généralement de rotation, à l'intérieur de la cavité (13).

7. Appareil de l'une quelconque des revendications précédentes, muni d'un conduit de gaz (156) à travers lequel un gaz peut être introduit dans la cavité (13), et éventuellement d'un conduit d'évacuation de gaz à travers lequel le gaz peut être évacué de la cavité (13), et éventuellement d'une source de rayonnement visible ou ultraviolet, agencée de manière à fournir le rayonnement visible ou ultraviolet à l'intérieur de la cavité (13).

8. Appareil de l'une quelconque de la revendication 1 ou 2, dans lequel la source (14) est située à l'extérieur de la cavité (13) et un conduit est prévu pour le plasma gazeux ionisé (9) ou pour les espèces réactives générées par le plasma gazeux ionisé (9) de la source (14) à la cavité (13), l'appareil comprenant éventuellement une source d'eau en communication avec la cavité (13), telle qu'une source d'eau activée par plasma.

9. Procédé de traitement d'un matériau, comprenant le placement du matériau à l'intérieur de la cavité (13) d'un appareil selon l'une quelconque des revendications précédentes, puis le fait d'amener la source (14) de plasma gazeux ionisé (9) à produire un plasma gazeux ionisé (9) tout en amenant également l'appareil d'agitation à agiter le matériau.

10. Procédé de la revendication 9, dans lequel le plasma gazeux ionisé (9) génère des espèces réactives qui s'accumulent dans la cavité (13), dans lesquelles les espèces réactives comprennent éventuellement au moins l'un parmi de l'ozone, de l'oxyde nitrique (NO), du peroxyde d'hydrogène (H₂O₂), des radicaux hydroxyles (OH), des ions hydronium (H⁺), des ions nitrate (NO₃⁻), des ions nitrite (NO₂⁻) et des atomes d'oxygène (O).

11. Procédé de la revendication 9 ou 10, dans lequel le matériau comprend des graines.

12. Procédé de la revendication 9 ou 10, dans lequel le matériau comprend un matériau granulaire, des billes de plastique, de la biomasse, des eaux usées, du lait ou de l'eau, ou tout autre matériau contaminé par des polluants.

13. Procédé selon la revendication 11, dans lequel le procédé aboutit à la désinfection et/ou au nettoyage des graines et/ou à la promotion de la germination des graines, généralement dans lequel la désinfection et/ou le nettoyage des graines et la promotion de la germination des graines se produisent au cours de la même période pendant laquelle les graines ne sont pas retirées de la cavité (13).

14. Procédé de la revendication 11 ou 13, dans lequel le procédé aboutit à la destruction de virus, de bactéries, de champignons ou de spores fongiques présents sur les graines.

15. Procédé de l'une quelconque des revendications 9 à 14, comprenant l'introduction d'un gaz dans la cavité (13) par le biais d'un conduit de gaz, et éventuellement l'évacuation du gaz par le conduit d'évacuation de gaz et/ou, lorsque l'appareil comprend une source d'eau en communication avec la cavité (13), l'ajout d'eau dans la cavité (13) afin de générer de l'eau activée par plasma à l'intérieur de la cavité (13).
